# EUROPÄISCHE PATENTSCHRIFT

(11) **EP 4 096 737 B1**
(45) Veröffentlichungstag und Bekanntmachung des Hinweises auf die Patenterteilung: **14.05.2025**
(21) Anmeldenummer: 21703379.4
(22) Anmeldetag: 26.01.2021
(51) Int. Cl.: A61M 1/16

(54) **VORRICHTUNG UND VERFAHREN ZUR HERSTELLUNG VON DIALYSAT**
SYSTEM AND METHOD FOR GENERATING DIASYLATE
DISPOSITIF ET MÉTHODE POUR PRÉPARER DU DIALYSAT

(30) Priorität: 28.01.2020 DE 102020101969; 12.03.2020 DE 102020106749; 12.03.2020 DE 102020106751
(43) Veröffentlichungstag der Anmeldung: 07.12.2022
(73) Patentinhaber: Fresenius Medical Care Deutschland GmbH, 61352 Bad Homburg (DE)
(72) Erfinder: MARTERSTOCK, Stefan, Konrad, 97337 Dettelbach (DE); KELLER, Torsten, 66606 St. Wendel (DE); GLASER, Benedict, 97421 Schweinfurt (DE)
(74) Vertreter: Herrmann, Uwe
(86) Internationale Anmeldenummer: PCT/EP2021/051722
(87) Internationale Veröffentlichungsnummer: WO 2021/151874

(56) Entgegenhaltungen:
- EP-A1- 3 187 211
- EP-A1- 3 272 375
- WO-A1-2014/128293
- WO-A2-2009/083011

## Beschreibung

Die vorliegende Erfindung betrifft eine Vorrichtung und ein Verfahren zur Herstellung von Dialysat.

Aus dem Stand der Technik ist es bekannt, Dialysegeräte mit gebrauchsfertigem Dialysat, d.h. mit einer gebrauchsfertigen Dialyselösung, z.B. aus einem Leitungssystem zu versorgen, das mit einer zentralen Einrichtung zur Herstellung des Dialysat verbunden ist und das im Allgemeinen ausgebildet ist, eine Mehrzahl von Dialysegeräten mit Dialysat zu versorgen.

Des Weiteren ist es aus dem Stand der Technik bekannt, das Dialysat am Dialysegerät selbst, d.h. dezentral herzustellen. Dazu kann eine RO-Anlage (RO = Revers-Osmose) zur Herstellung von Reinstwasser vorgesehen sein, die Bestandteil des Dialysegerätes ist oder als eine gesonderte Einheit ausgeführt sein kann. Das Reinstwasser wird in dem Dialysegerät mit einem oder mehreren Konzentraten gemischt, um ein gebrauchsfertiges Dialysat zur Behandlung des Patienten zu erhalten. Bei der Herstellung von Reinstwasser mittels des RO-Prozesses besteht ein Nachteil darin, dass der Prozess hohe Drücke im Bereich von 6 bis 15 bar erfordert, was entsprechend energieaufwändig ist.

Die Druckschriften EP3272375A1 und EP3187211A1 offenbaren jeweils eine Vorrichtung und ein Verfahren zur Erzeugung eines Dialysats zur Dialyse. Aus der WO2014/128293A1 ist ein Wasserextraktionssystem bekannt. Die WO2009/083011A2 offenbart ein Verfahren zur regenierung von Diaylseflüssigkeit.

Die Druckschriften EP3272375A1 und EP3187211A1offenbaren jeweils eine Vorrichtung und ein Verfahren zur Erzeugung eines Dialysats zur Dialyse. Aus der WO2014/128293A1 ist ein Wasserextraktionssystem bekannt. Die WO2009/083011A2 offenbart ein Verfahren zur Rregenierung von Dialyseflüssigkeit.

Der vorliegenden Erfindung liegt die Aufgabe zugrunde, eine Vorrichtung und ein Verfahren bereitzustellen, mittels derer/dessen eine energieeffiziente Dialysatherstellung möglich ist.

Diese Aufgabe wird durch eine Vorrichtung mit den Merkmalen des Anspruchs 1 sowie durch ein Verfahren mit den Merkmalen des Anspruchs 12 gelöst.

Danach ist vorgesehen, dass die Vorrichtung einen ersten Teil und einen zweiten, als Kreislauf ausgebildeten Teil aufweist, wobei der erste Teil einen Wasseranschluss oder einen Wasserbehälter sowie die Primärseite eines Filters umfasst, wobei der Filter ausgebildet ist, um aus dem Wasser gereinigtes Wasser durch Forward-Osmose herzustellen, und wobei der zweite Teil die Sekundärseite des Filters, ein Reservoir, eine Filtratleitung, die von der Sekundärseite des Filters zu dem Reservoir führt, und eine von dem Reservoir zu der Sekundärseite des Filters führende Leitung umfasst, wobei der Behälter über Mittel zum Verbinden des Behälters mit einem Dialysegerät verfügt.

Vorzugsweise ist vorgesehen, dass aus dem Wasserbehälter bzw. aus dem Wasseranschluss Roh-Wasser bzw. Leitungswasser bezogen wird.

In dem Behälter können sich ein oder mehrere Konzentrate befinden, aus denen durch Verdünnung oder Auflösung in Permeat ein Dialysat hergestellt werden kann.

Der Begriff "Dialysat" umfasst im Rahmen der vorliegenden Erfindung sowohl eine gebrauchsfertige Dialyselösung als auch einen oder mehrere Bestandteile von dieser.

Der vorliegenden Erfindung liegt somit der Gedanke zugrunde, die unterschiedliche Elektrolytkonzentration zwischen Roh-Wasser und Dialysat/Konzentrat und dem damit verbundenen osmotischen Druck zu nutzen, um mit Hilfe einer geeigneten Forward-Osmose Membran des Filters Dialysat herzustellen. Die Forward-Osmose wird im Folgenden auch kurz FO genannt.

Für die Herstellung von Dialysat wird das Permeat nach der Herstellung mit einem oder mehreren Konzentraten gemischt, bis eine physiologische bzw. die gewünschte Elektrolytkonzentration erreicht ist. Vorzugsweise befindet sich zumindest eines der Konzentrate in dem Reservoir. Denkbar ist es, dass zumindest ein Konzentrat (oder alle Konzentrate) dem zweiten Teil der Vorrichtung über eine Konzentratleitung zugeführt wird.

Die Stoffkonzentration an Elektrolyten (und damit die Leitfähigkeit als Summenparameter für die Elektrolytkonzentration) ist im Dialysat bzw. im Dialysatkonzentrat deutlich höher als in Leitungswasser. So beträgt die elektrische Leitfähigkeit von Trinkwasser (laut deutscher Trinkwasserverordnung): max. 2,79 mS/cm, die elektrische Leitfähigkeit von Dialysat üblicherweise 12 bis 16 mS/cm. Dieser (die Erfindung nicht beschränkende) Unterschied in der Elektrolytkonzentration führt zu einem osmotischen Druckgradient, der erfindungsgemäß für den Prozess der Dialysatherstellung durch FO ausgenutzt wird.

Vorzugsweise wird das Wasser, bei dem es sich vorzugsweise um Leitungswasser handelt, zur Erzeugung von Dialysat oder -konzentrat (vorzugsweise auf 37°C) erwärmt. Geschieht dies direkt am Anfang des Prozesses, verbessert sich der Wirkungsgrad des FO-Prozess, da der osmotischen Druckgradient direkt temperaturabhängig ist.

Ein wesentlicher Bestandteil der Erfindung ist der Einsatz der Forward-Osmose zur Dialysataufbereitung. Hier steht nicht die Verwendung von gebrauchtem Dialysat im Vordergrund, sondern die Aufbereitung von Rohwasser, z.B. aus der Hausversorgung. Die FO-Membran ist somit vorzugsweise auch Sterilbarriere, so dass eine gute Heimdialyse sowohl für HD (Hämodialyse) als auch für PD (Peritonealdialyse) aus Konzentraten (trocken, flüssig etc.) möglich ist.

Vorzugsweise wird das Konzentrat bzw. das Dialysat auf der Sekundärseite solange im Kreislauf geführt, bis die gewünschte Endkonzentration, -leitfähigkeit etc. erreicht ist.

Bei dem zweiten Teil der Vorrichtung handelt es sich um einen Kreislauf. In einer bevorzugten Ausgestaltung der Erfindung ist auch der erste Teil der Vorrichtung als Kreislauf ausgebildet, d.h. das Wasser wird im Kreislauf an der Primärseite des Filters und das Dialysat oder das Dialysatkonzentrat wird im Kreislauf an der Sekundärseite des Filters vorbeigeführt.

Mit einer Vorrichtung gemäß der vorliegenden Erfindung können beispielsweise eine oder besser zwei kleine Kammern bzw. Behälter mit gebrauchsfertigem Dialysat gefüllt werden, so dass eine Kammer immer für die Entnahme von Dialysat zur Verfügung steht. Diese Vorrichtung wird im Rahmen der Erfindung auch "Mikro-Batch" genannt. Es kann aber auch vorgesehen sein, einen großen Vorrat von beispielsweise 2-5 I (typisch für PD) oder 70-100 I (typisch für HD), beispielsweise in einem Behälter, insbesondere Beutel anzulegen. Eine solche Vorrichtung bzw. Verfahren wird im Rahmen der Erfindung auch "Makro-Batch" genannt.

Ein FO-Prozess nutzt den osmotischen Druckgradienten zur Filtration von Wasser, vorzugsweise von Leitungswasser. Die energieaufwändige Produktion von Permeat durch RO findet erfindungsgemäß nicht statt und es wird direkt Dialysat bzw. -konzentrat hergestellt. Da der Aufbereitungsprozess vorzugsweise unmittelbar vor der Verwendung des Fluids geschieht, d.h. sich kein Rohrleitungsnetz dazwischen befindet, vereinfacht sich der Aufwand zur Einhaltung der nötigen Hygiene. Durch eine stetige Miniaturisierung sind zudem neue Möglichkeiten hinsichtlich Portabilität denkbar. Zudem ist der Prozess deutlich leiser, als der einer RO-Anlage mit dazugehöriger Pumpe zur Druckerzeugung.

Vorzugsweise handelt es sich bei dem Behälter um einen Beutel, der teilweise oder insgesamt flexible Wandungen aufweist. Auch ein Behälter, der insgesamt oder teilweise starre Wandungen aufweist, wie z.B. eine Kartusche, ist denkbar und von der Erfindung mit umfasst.

Erfindungsgemäß ist vorgesehen, dass mehrere, vorzugsweise zwei Behälter vorgesehen sind und dass eine Ventilanordnung vorhanden ist, die ausgebildet ist, die Behälter abwechselnd dem zweiten Kreislauf zuzuschalten. Der jeweils nicht dem zweiten Kreislauf zugeschaltete Behälter kann entnommen und zur Verwendung bei der Dialyse eingesetzt werden. Gleichzeitig wird der andere Behälter mit dem gebrauchsfertigen Dialysat bzw. mit -konzentrat gefüllt.

Bei den genannten Mitteln des Behälters kann es sich um einen Konnektor oder um einen Schlauch oder um ein Anschluss für einen Schlauch oder um sonstige Mittel handeln, durch die eine Fluidverbindung zwischen dem Inneren des Behälters und einem Dialysegerät herstellbar ist. Mittels eines Konnektors ist der Behälter, der vorzugsweise als Beutel ausgebildet ist, vorzugsweise unmittelbar mit einem Gegenstück an einem Dialysegerät fixierbar. Auch ist es denkbar, dass der Behälter über einen Schlauch verfügt oder über einen Adapter für einen Schlauch, mittels dessen das gebrauchsfertige Dialysat bzw. Dialysatkonzentrat verwendet kann, wie z.B. im Bereich der Peritonealdialyse.

Bei dem sich vorzugsweise in dem Behälter befindlichen Konzentrat kann es sich beispielsweise um ein Bicarbonat-Konzentrat und/oder um ein saures Konzentrat handeln, das zur Herstellung von Dialysat ausgebildet ist.

Das Konzentrat kann in dem Behälter z.B. als Pulver, Granulat, Slurry oder in flüssiger Form vorliegen.

In dem ersten Teil und/oder in dem zweiten Teil der Vorrichtung, der auch als "zweiter Kreislauf" bezeichnet wird, ist vorzugsweise eine Pumpe angeordnet. Befindet sich die Pumpe in dem ersten Teil kann mittels der Pumpe ein ausreichend hoher Druck auf der Primärseite des Filters erzeugt werden. Die Pumpe auf der Sekundärseite hat den Vorteil, dass das Dialysat bzw. das Dialysatkonzentrat wiederholt so oft an der Filtermembran vorbeigeführt werden kann, bis die gewünsche Konzentration bzw. Leitfähigkeit etc. erreicht ist.

Um den Endzeitpunkt der Herstellung des Dialysats bzw. des Dialysatkonzentrats zu erfassen, kann in dem zweiten Kreislauf ein Sensor, vorzugsweise eine Leitfähigkeitsmesszelle angeordnet sein. Weist diese einen Messwert auf, der in einem Sollwertbereich liegt, kann die Herstellung des Dialysats bzw. des -konzentrats als beendet angesehen und der Behälter zur Verwendung in der Dialysebehandlung entnommen werden.

Denkbar ist es, dass in den zweiten Kreislauf eine Konzentratleitung einmündet, die ihrerseits mit einem Reservoir für Dialysatkonzentrat verbunden ist, so dass mittels der Konzentratleitung ein weiteres Konzentrat in den zweiten Kreislauf einführbar ist. Dies ist für den Fall sinnvoll, dass sich in dem Behälter nicht alle benötigten Konzentrate befinden, sondern nur ein Teil von diesen.

Der Behälter kann genau ein Kompartiment aufweisen, in dem sich ein oder mehrere Konzentrate befinden, die im Rahmen der vorliegenden Erfindung mittels des Permeats gelöst werden. Auch ist es denkbar, dass der Behälter mehrere Kompartimente aufweist, in denen jeweils ein oder mehrere Konzentrate vorliegen. Dabei ist es möglich, dass die Kompartimente so angeordnet und ausgebildet sind, dass diese zeitlich gestaffelt öffnen, so dass zeitlich gestaffelt bestimmte Osmolaritäten vorliegen.

Die vorliegende Erfindung betrifft des Weiteren ein Verfahren zur Herstellung einer Dialysat mit einer Vorrichtung nach einem der Ansprüche 1 bis 12, wobei der Primärseite des Filters Wasser, vorzugsweise Leitungswasser zugeführt wird, wobei das Permeat durch Forward-Osmose der Sekundärseite zugeführt wird und wobei der Sekundärseite des Filters aus dem Behälter und/oder aus einer sonstigen Quelle ein Dialysat oder ein Dialysatkonzentrat zugeführt wird, das mit dem Permeat gemischt wird.

Vorzugsweise ist vorgesehen, dass das Dialysat oder das Dialysatkonzentrat auf der Sekundärseite im Kreislauf gefördert wird, bis die Leitfähigkeit und/oder eine Konzentration oder ein sonstiger damit korrelierter Parameter einem Sollwert entspricht oder in einem Sollwertbereich liegt.

Denkbar ist es, dass im Falle des Vorliegens mehrerer Behälter ein Behälter in dem zweiten Kreislauf mit dem Dialysat oder mit dem Dialysatkonzentrat gefüllt wird und der andere Behälter zur Verwendung in einem Dialysegerät, d.h. im Rahmen einer Dialysebehandlung entleert wird.

Vorteilhaft ist es, wenn zur Erhöhung des osmotischen Drucks auf der Sekundärseite des Filters ein physiologisch verträglicher Stoff, insbesondere Glucose, oder ein vor der Verwendung als Dialyelösung abzuscheidener Stoff, insbesondere magnetische Nano Eisenpartikel zugegeben werden.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: ein schematisches Flussbild einer Vorrichtung gemäß der Erfindung in einer ersten Ausführungsform (Mikro-Batch),
- Figur 2:: ein schematisches Flussbild einer Vorrichtung gemäß der Erfindung in einer zweiten Ausführungsform (Makro-Batch) und
- Figur 3:: ein schematisches Flussbild einer Vorrichtung gemäß der Erfindung in einer dritten Ausführungsform (2 x Mikro-Batch).

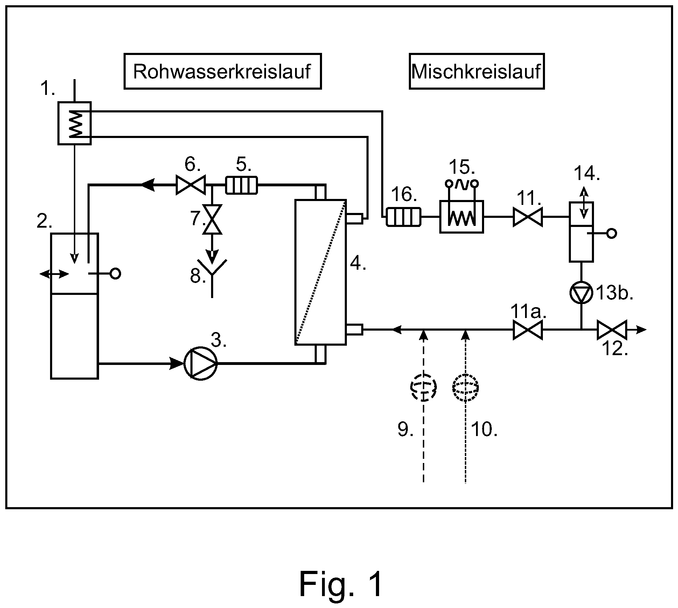

Figur 1 zeigt mit dem Bezugszeichen 1 einen Wärmetauscher am Rohwasserzulauf. Die Erwärmung des dem Filter 4 zugeführten Rohwassers erhöht die Effektivität des Osmose-Prozesses. Mit dem Bezugszeichen 2 ist die Rohwasser-Eingangskammer gekennzeichnet, die eine Belüftung und/oder einen Niveausensor aufweist.

Wie dies aus Figur 1 hervorgeht, umfasst die Vorrichtung einen ersten Teil in Form des Rohwasserkreislaufs und einen zweiten Teil in Form des Mischkreislaufs.

Das Bezugszeichen 3 kennzeichnet die Zirkulationspumpe im Rohwasserkreislauf, d.h. in dem erfindungsgemäß ersten Kreislauf. Mit 4 ist der FO-Filter gekennzeichnet, 5 kennzeichnet die Leitfähigkeits-Temperatur-Messzelle für Roh-Wasser und "Back-Flow" Kontrolle. Das Bezugszeichen 6 kennzeichnet das Rohwasser-Zirkulationsventil und 7 das Flush-Ventil zum Spülen des Filters 4.

Mit 8 ist der Abfluss und mit 9 eine A-Konzentrat Pumpe (Dialyse Konzentrat / Säure Konzentrat) (A-Konzentrat kann auch später zugegeben werden) und mit 10 eine B-Konzentrat Pumpe (Dialyse Konzentrat / Bikarbonat Konzentrat) gekennzeichnet.

Das Bezugszeichen 11 kennzeichnet ein Mischkreislauf-Zirkulationsventil und 11a ein Absperrventil, das mittels einer Steuereinheit so angesteuert wird, dass es schließt, sobald die Zielleitfähigkeit erreicht wurde.

Das Bezugszeichen 12 ist das Dialysat-Entnahme-Ventil und 13b die Zirkulationspumpe im Mischkreislauf, d.h im erfindungsgemäß zweiten Kreislauf.

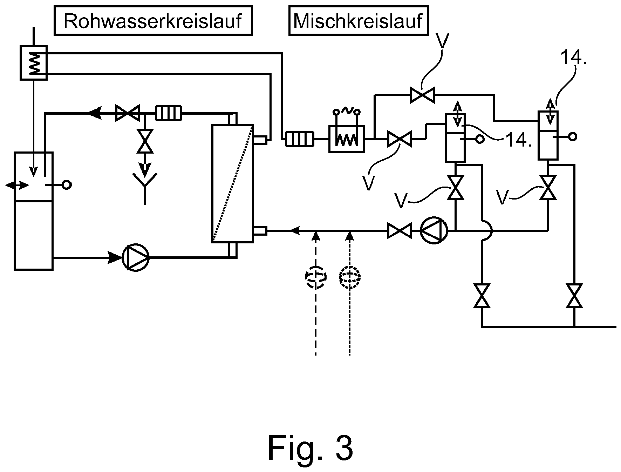

Das Bezugszeichen 14 kenneichnet einen Konzentrat-Beutel, der als Sammelkammer dient und mit Belüftung und Niveausensor (hier wird das Filtrat kumuliert) ausgeführt sein kann. Bevorzugt werden zwei parallele Sammelkammern, vorzugsweise Konzentrat-Beutel vorgesehen, wie dies aus Figur 3 hervorgeht. Damit kann eine Kammer immer mit Dialysat gefüllt und die jeweils andere Kammer immer entleert bzw. im Rahmen der Dialysebehandlung verwendet werden.

Das Bezugszeichen 15 ist eine Heizung, die auch an der Position des Wärmetauschers angeordnet sein kann, das Bezugszeichen 16 ist eine Leitfähigkeits-Temperatur-Messzelle zur Überwachung der Dialysat Qualität und Zusammensetzung (Filtrat + Bikarbonat-Mischungsverhältnis bzw. Filtrat + Bikarbonat + Säure Konzentrat - Mischungsverhältnis). Alternativ kann eine zweite Messzelle zur Nachregelung des B-Konzentrats verwendet werden.

Der Ablauf zur Herstellung von Dialysat oder Dialysekonzentrat gestaltet sich exemplarisch wie folgt:

### 0. (Initiales Vorbereiten)

Der FO-Filter 4 wird auf der Rohwasser-Seite, d.h. auf der Seite des ersten Kreislaufs gefüllt mit Roh-Wasser (Leitungswasser).

Der FO-Filter 4 wird auf der Filtrat-Seite mit physiologischer Lösung gefüllt oder mit Rohwasser oder Permeat (= Filtrat) wird durch geringen Überdruck auf der Rohwasserseite auf die Filtratseite gedrückt.

### 1. Konzentrat Zugabe in den Mischkreislauf

Beispiel: Volumen im Mischkreislauf: 800 ml (400 ml im Filter / 400 ml im Kreislauf) Zugabe an Konzentrat: 11,4 ml A-Konzentrat und 14 ml B-Konzentrat.

### 2. Permeatzugabe in den Mischkreislauf

Durch die FO Membran strömt Permeat (Filtrat) nach, bis das richtige Mischungsverhältnis hergestellt ist. Die ist relevant für den osmotischen Druck und damit für den Permeatfluss durch die Membran.

Kontrolle des Mischungsverhältnisses (Ziel Na-Konzentration im Dialysat typisch 138 mmol/L) durch Messung der der Dialysat-Leitfähigkeit (typisch 12 bis 16 mS/cm).

### 3. Entnahme des fertigen Dialysats

Neuer Zyklus startet mit der Zugabe von Konzentrat (1 → 2 →3)

Der gesamte Vorgang kann auch als kontinuierlicher Prozess mit kontinuierlicher Konzentrat-Zugabe, Permeat-Produktion und Dialysat-Entnahme ausgelegt werden.

Zudem ist es möglich, eine zweite Sammelkammer bzw. -behälter und vorzugsweise Beutel einzubauen, die alternierend zur ersten Kammer befüllt wird. Somit kann eine Dialysat Entnahme aus einer Kammer erfolgen, während die andere Kammer befüllt wird. Damit ist eine quasi kontinuierliche Dialysat Produktion sichergestellt.

Als Optionen zur Steigung der Osmolarität auf der Produkt Seite des Filters 4 sind vorzugsweise zu nennen:
Um den osmotischen Gradienten zwischen Leitungswasser und Dialysat zu erhöhen und damit die Leistungsfähigkeit des Osmose Prozesses, ist es möglich auf der der Produktseite, d.h. in dem zweiten Kreislauf:
a. Einen physiologisch verträglichen Zusatzstoff zuzugeben. Glukose wäre hier ein möglicher Stoff, der heute schon in Konzentrationen bis zu 1 g/l in HD-Dialysat enthalten ist.
b. Einen Stoff zuzugeben, der nicht im Dialysat verbleibt, sondern vorher physikalisch oder chemisch abgeschieden wird (oder von Dialysator zurückgehalten wird). Magnetische Nano Eisenpartikel wären hierfür geeignet. Die Partikel werden vor dem Filter zugegeben und nach dem Filter mit Hilfe eines Magnetfelds abgeschieden.

Auch eine Rückführung des verbrauchten Dialysats in die Kammer 2 wäre denkbar, um eine Wiederaufbereitung vorzunehmen und so Energie und Wasser einzusparen.

Ebenfalls vorstellbar ist es, den FO-Prozess in dem Filter 4 durch einen zusätzlichen, hydraulischen Druck oder Unterdruck einer Pumpe zu unterstützen.

Als ergänzende Idee ist Folgendes zu nennen: Den Forward-Osmose Filter 4 im Betrieb zu testen, um sicherzustellen, dass die Rückhaltefunktion noch gewährleistet ist.

Mit dem Einsatz der Forward Osmosis Technik in sensiblen Bereichen, wie der Dialysetechnik wird es notwendig, die korrekte Funktionsweise der FO-Membran sicherzustellen oder zumindest zu überwachen.

Als Mögliche Lösungen sind zu nennen:
1. Messung des sich einstellenden osmotischen Drucks, bei korrekter Funktion der FO-Membran. Sollte die Membran in Ordnung sein, so wird sich ein transmembraner Druck über der FO-Membran (der osmotische Druck) aufgebaut. Mit Ventilen können die Zu- und Abgänge des FO-Filters gesperrt werden und mit einem Drucksensor der osmotische Druck gemessen werden. Zuvor wird der Filter entsprechend gefüllt (mit Konzentrat auf der Sekundärseite und mit z.B. Leitungswasser auf der Primärseite). Der sich einstellende Druck muss dann über einen gewissen Zeitraum konstant bleiben, anderenfalls ist anzunehmen, dass eine direkte Mischung der normalerweise durch die FO-Membran getrennten Flüssigkeiten stattfindet.
2. Priming des FO-Filters durch Abdrücken von Leitungswasser auf die Permeatseite und anschließend Messung der Leitfähigkeit des Permeats.
3. Bestimmung der zugeflossenen und der entnommenen Fluidmengen (am FO-Filter) sowie deren Leitfähigkeit. Mit den Werten kann dann eine Plausibilisierung der Verdünnung durch den FO-Prozess durchgeführt werden.
4. Filter sekundärseitig mit Luft füllen. Primärseitig mit Flüssigkeit beaufschlagen und den Druck messen, ab dem der Filter "durchbricht", d.h. Flüssigkeit auf die Sekundärseite übertritt (sog. Bubble-Point Test).

Nach dem Makro-Batch Ansatz erfolgt die Forward-Osmosis gestützte Dialysat-Erzeugung wie folgt:
Die Lösung wird in einen oder mehreren Batch(-es) hergestellt. Der Batch kann z.B. ein Beutel sein, der die Konzentrate zum Herstellen der Lösung in fester oder flüssiger Form enthält. Vorteilhaft ist es, wenn die Menge an Dialysat zur Durchführung einer Dialysebehandlung ausreicht (60L bis 250 L).

Eine Beschreibung einer möglichen Ausführungsform des Verfahrens ist wie folgt:

### Primärseite

Die Primärseite ("Feed" Seite) der FO-Membran ist mit einer Rohwasser-Quelle verbunden (Leistungswasser-Anschluss). Der Druck der Rohwasser-Quellen oder eine separate Druckerhöhungseinrichtung (Pumpe oder hydrostatischer Druckspeicher) sorgen dafür, dass die Primärseite Rohwasser überströmt wird. Die gleiche Druckquellen kann auch verwendet werden, um die Primärseite initial zu füllen.

### Sekundärseite / Sekundärkreislauf

Die Sekundärseite ("Produkt" Seite) der FO-Membran ist mit dem Batch, d.h. mit dem Behälter, vorzugsweise mit dem Beutel verbunden. Eine separate Druckerhöhungseinrichtung (Pumpe oder hydrostatischer Druckspeicher) sorgen dafür, dass die Sekundärseite Lösung überströmt wird.

Vorzugweise wird die Lösung immer wieder an der der FO-Membran entlanggeführt. D.h. der Batch ist über einen Kreislauf mit dem FO-Filter verbunden.

### Primen (Füllen)

Zu Beginn befinden sich nur die Konzentrate in trockener Form oder gering verdünnt im Batch (als Pulver, Granulat, "Slurry" oder in flüssiger Form). Die Konzentrate werden mit wenig Lösungsmittel (i.d.R. Leitungswasser) gelöst/verdünnt. Die Verdünnung ist dabei so vorzunehmen, dass die verwendete FO-Membran durch die immer noch stark erhöhte Elektrolytkonzentration keinen Schaden nimmt (keine Auskristallisation an der Membran o.ä.). Alle Konzentrate können von Anfang an in der Konzentrat-Lösung vorliegen oder erst mit der Zeit dem Batch zugegeben werden.

Werden die Konzentrate erst zeitlich verzögert zugegeben, kann der osmotische Druckgradient in einem für die Effektivität der FO-Membran optimalen Bereich gehalten werden.

Möglichkeiten der initialen Verdünnung/Lösung der Konzentrate und zum Primen (Füllen) der Sekundärseite:
- Filter ist Vorgefüllt und/oder Batch ist ausreichend vorgefüllt um den Prozess zu starten und die Sekundärseite/ Sekundärkreislauf zu füllen
- Primen (Füllen) der Sekundärseite/ Sekundärkreislauf durch Unterdruck erzeugen → Pumpe auf der Sekundärseite saugt Filtrat an und füllt den Batchbag und den Sekundärseite des Filters (Kontrolle des Transmembrandrucks durch Drucksensor).
- Primen (Füllen) der Sekundärseite/ Sekundärkreislauf durch Überdruck auf der Primärseite → Druckpquelle Primärseite drückt Filtrat und füllt den Batchbag und den Sekundärseite des Filters (Kontrolle des Transmembrandrucks durch Drucksensor)
- Flüssigkeit zum Primen des Filters aus dem vorhergehenden Abfüllprozess als Priming-Lösung verwenden

Möglich ist es, initial nur die Filtrat Seite mit minimalem d.h. reduziertem Kreislauf zu betreiben/ füllen -> Verkleinertes Sekundärvolumen (geeignete, kleine Beutelform, FO-Filter mit reduzierten Sekundärvolumen.

Spätere Zugabe von Wasser z.B. durch Volumen aus FO-Membran oder durch manuelle Zugabestelle am Batch. Ziel: Geringeres Priming-Volumen

### Herstellung der Lösung

Die konzentrierte Lösung wird an der Sekundärseite der FO-Membran vorbeigeleitet. Der hohe osmotische Druckgradient zwischen Rohwasser und Lösung führt zu einem anhaltenden Filtratfluss über die FO-Membran in den Batch. Bei einem geschlossenen Batch nehmen der osmotische Druckgradient und damit die Produktion von Permeat mit der Zeit ab. Es bleibt jedoch bis zum Erreichen einer, für eine physioloschen Lösung typischen, Elektrolytkonzentration ein Gradient erhalten (Elektrolytkonzentration ca. 0,15 Mol/L in fertigem Dialysat).

Stopp der Herstellung:
- Volumensteifes System wird aufgefüllt -> Zielvolumen wird erreicht / statischer Druck steigt an → Druck messen oder der Prozess kommt von allein zum Erliegen (p_statisch = p_osmose)
- Abschaltung über LF (Leitfähigkeit) oder LF nur Schutzsystem
- Abschaltung über Gewichtsermittlung
- Zeitgesteuert
- TMP (Transmembranen Druck) messen / Filtrat-Fluss messen
- der Prozess endet, sobald das Mischungsverhältnis von A-Konzentrat zu Permeat z.B. 1:35 beträgt (= Mischungsverhältnis für fertiges Konzentrat).
- Tank (volumensteif)

Vorteile der vorliegenden Erfindung sind in bevorzugter Ausführung:
- Ein FO-Prozess (insbesondere FO-Membranen mit Auqaporin-Technologie) nutzt den osmotischen Druckgradient zur Filtration von Leitungswasser. Die energieaufwändige Produktion von Permeat wird "ausgelassen" und es wird direkt Dialysat hergestellt. Dennoch werden vergleichsweise hohe Filtrationsraten erreicht:
   FO-Filter (z.B. Aquaporin Inside HFFO2): 22,60 Liter/m²/Stunde (25°C / 5,8% NaCl Lösung vs. Tap water) RO-Filter z.B. 50 Liter/m²/Stunde bei 15 bar
- Da der Aufbereitungsprozess unmittelbar vor der Verwendung des Fluids geschieht, d.h. kein Rohrleitungsnetz dazwischen ist, vereinfacht sich der Aufwand zur Einhaltung der nötigen Hygiene. Durch eine stetige Miniaturisierung sind zudem neue Möglichkeiten hinsichtlich Portabilität denkbar.
- Bei einem FO-Prozess wird die energieaufwändige Produktion von Filtrat (Permeat) auslassen und direkt physiologische Lösung (Dialysat) herstellt.
- Die Produktion von Permeat über eine FO-Membran, im Vergleich allen genannten Verfahren, technisch weniger aufwändig (nur eine FO-Membran anstatt mehrere Adsorber Kartuschen, keine (für hohe Drücke ausgelegte) Rohrleitungssysteme) und eignet sich daher für Heimsysteme, wie sie bei der PD-Dialyse üblich sind.
- Das gesamte System ist damit raum-, und kostensparender, weniger komplex, besser zu reinigen.
- Zudem ist eine bessere Filtrat-Qualität im Vergleich zur Adsorbertechnik zu erwarten, da die Rückhalteraten der FO-Membranen, an die Rückhalteraten von RO-Membranen fast heranreichen (RO-Technlogie realisiert derzeit die maximal Rückhalterate bei Filtrationsprozessen) □ Wichtig bei der Herstellung hygienisch besonders kritischer PD-Lösungen, die direkt in den Bauraum eines Patienten infiltriert werden.
- Versand von Trockenkonzentratbeuteln oder hoch konzentrierten Konzentraten anstatt der aktuellen gebrauchsfertigen Flüssig-Lösungen (niedrigeres Transportgewicht, bessere Haltbarkeit und Keimfreiheit)
- Insgesamt eignet sich die Technologie zur dezentralen, bedarfsgerechten Herstellung physiologischer & hygienischer Lösungen
- Prozess ist deutlich leiser, als eine RO-Anlage mit dazugehöriger Pumpe zur Druckerzeugung → besser geeignet für das Heimumfeld.

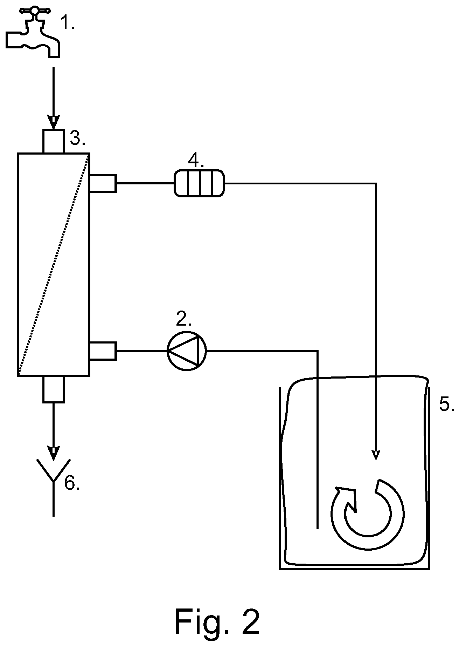

Figur 2 zeigt eine Vorrichtung in schematischer Ansicht zur Herstellung eines Makro-Batches:
Das Bezugszeichen 1 zeigt den Wasser Anschluss, 2 die Zirkulationspumpe des Mischkreislaufs, 3 den FO-Filter, 4 die Leitfähigkeits-Temperatur-Messzelle für Roh-Wasser und "Back-Flow" Kontrolle (könnte auch zwischen 5 und 2 in der Saugleitung angeordnet sein), 5 den Behälter, vorzugsweise Beutel mit Konzentrat(en) und 22 den Abfluss. Wie aus Figur 2 ersichtlich, ist in diesem Ausführungsbeispiel der erste Teil der Vorrichtung nicht als Kreislauf ausgeführt.

Der FO-Filter ist vorzugsweise Disposable/Semi-Disposable und wird nach Bedarf (festes Intervall, nach bestimmten Volumen an Filtrat, "Bubble Point Test" / "Druckhaltetest fällt durch) ausgetauscht.

Als Optionen / Erweiterungsmöglichkeiten sind zu nennen:
- Luftabscheidung in Kreislauf, um Luft am Filter zu vermeiden
- Druckerhöhung auf der Rohwasserseite, um den Rohwasserfluss zu erhöhen oder den FO-Prozess mit einem hydrostatischen Druck zu überlagern (→ Steigerung der Filtrationsrate)
- Kombination mit Elektrodialyse zur Erhöhung der (volumetrischen) Konzentration der frei beweglichen Elektrolyte durch Anlegen eines elektrischen Feldes → Erhöhte Konzentration der Elektrolyte am Filter
- Einbringen eines physiologisch neutralen Stoffes zur Erhöhung des osmotischen Druckes
- Einbringen eines, in einen weiteren Schritt abtrennbaren Stoff (FO-Agent) - Nachträgliche Trennung des Agents vom Produkt (wie heute üblich) z.B. über thermischen Prozess, Filtration oder durch Abscheidung im Magnetfeld (bei Verwendung eines Ferrofluid als Agent).
- Wiederverwendung von bereits verbrauchtem Dialysat, durch Zuführung des gebrauchten Dialysats auf der Primärseite / Beutel-Entleerung -> Abfallgewicht und Volumen geringer.
- Gebrauchtes Dialysat wird während der gesamten Dialyse wiederverwendet
- Gebrauchtes Dialysat wird zum Start des Prozesses verwendet (Sekundärkreislauf füllen)
- Überwachung des Transmembrandruck (Grenzwerte spezifisch für FO Filter), um eine Schädigung des FO-Filter zu vermeiden (→ wichtig bei Aquaporin-Filtern)
- FO-Membran-Tests zur Überwachung der Integrität des Filters
- Befüllung/Entleerung mehrerer Beutel gleichzeitig.
- Überwachung der Leitfähigkeit im Batch, in der Ansaugleitung vor dem FO-Filter oder nach dem FO-Filter zur Steuerung des Filtrat Produktionsprozess
- Heizung und/oder Wärmetauscher im Primär- oder Sekundärkreislauf (bevorzugt), um das Rohwasser/Filtrat auf eine für den FO-Prozessoptimale Temperatur zu bringen (25 bis 50 °C)
- Zusätzlicher Ultrafilter in der Zuleitung vor dem FO-Kreislauf, um hohe mikrobiologische, chemische oder physikalische Verunreinigungen zurückzuhalten und damit das "Fouling" des FO-Filters zu reduzieren und höhere Standzeiten zu erzielen
- Zusätzlicher Ultrafilter nach dem FO-Kreislauf, um noch vorhandene mikrobiologische (Endotoxine), chemische oder physikalische Verunreinigungen zurückzuhalten
- Test des FO-Filters (z.B. Messung osmotischer Druck) vor und nach der Batch-Befüllung, um die Filterwirkung und damit die Validität des Batches sicherzustellen. Freigabe des Batch erst nach erfolgreichem Test (z.B.)
- Prinzipiell analog alle beschriebenen Ausführungen im Rahmen des Mirko-Batch-Ansatzes

Mögliche Anwendungsgebiete der Erfindung sind:
- HD-Lösung herstellen / Batch für eine Behandlung z.B. 70L
- PD-Lösung herstellen / Batch für eine Behandlung oder mehrere Beutel (z.B. 5 L-Beutel)
- Mobile Herstellung von NaCl-Lösung (nach Katastrophen, Notfällen oder bei schlechter Energieversorgung)
- Genius-Tank mit FO System befüllen, Akut-Beutel herstellen

Figur 3 zeigt eine im Wesentlichen Figur 1 entsprechende Vorrichtung, wobei allerdings zwei Behälter 14 vorgesehen sind, die durch Ventile V fluidisch von dem zweiten Kreislauf, d.h. von dem Mischkreislauf getrennt werden können oder diesem zugeschaltet werden können. Während der Herstellung der Lösung wird wechselseitig ein Behälter 14 dem Kreislauf zugeschaltet und der andere Behälter 14 entnommen und mit einem Dialysegerät, d.h. im Rahmen der Behandlung verwendet.

## Patentansprüche

1. Vorrichtung zur Herstellung von Dialysat, wobei die Vorrichtung einen ersten Teil und einen zweiten, als Kreislauf ausgebildeten Teil aufweist, wobei der erste Teil einen Wasseranschluss oder einen Wasserbehälter (2) sowie die Primärseite eines Filters (4) umfasst, wobei der Filter (4) ausgebildet ist, um aus dem Wasser gereinigtes Wasser durch Forward-Osmose herzustellen, und wobei der zweite Teil die Sekundärseite des Filters (4), ein Reservoir (14), eine Filtratleitung, die von der Sekundärseite des Filters (4) zu dem Reservoir (14) führt, und eine von dem Reservoir (14) zu der Sekundärseite des Filters (4) führende Leitung umfasst, wobei es sich bei dem Reservoir (14) um einen Behälter handelt, der über Mittel zum Verbinden des Behälters mit einem Dialysegerät verfügt, **dadurch gekennzeichnet, dass** mehrere Behälter vorgesehen sind und dass eine Ventilanordnung vorhanden ist, die ausgebildet ist, die mehreren Behälter abwechselnd in Fluidverbindung zu dem zweiten Teil der Vorrichtung zu schalten.

2. Vorrichtung nach Anspruch 1, **dadurch gekennzeichnet, dass** es sich bei dem Behälter um einen Beutel handelt, der insgesamt oder teilweise mit flexiblen Wandungen ausgeführt ist, oder um eine Kartusche handelt, die insgesamt oder teilweise mit starren Wandungen ausgführt ist.

3. Vorrichtung nach Anspruch 1 oder 2, **dadurch gekennzeichnet, dass** auch der erste Teil der Vorrichtung als Kreislauf ausgeführt ist.

4. Vorrichtung nach einem der vorhergehenden Ansprüche, **dadurch gekennzeichnet, dass** sich in dem Behälter Dialysatkonzentrat befindet.

5. Vorrichtung nach einem der vorhergehenden Ansprüche, **dadurch gekennzeichnet, dass** es sich bei den Mitteln des Behälters um einen Konnektor oder um einen Schlauch oder um ein Anschluss für einen Schlauch handelt.

6. Vorrichtung nach einem der vorhergehenden Ansprüche, **dadurch gekennzeichnet, dass** in dem Behälter ein Bicarbonat-Konzentrat und/oder ein saures Konzentrat vorliegt, das zur Herstellung von Dialysat ausgebildet ist.

7. Vorrichtung nach einem der vorhergehenden Ansprüche, daurch gekennzeichnet, dass in dem Behälter mehrere Kompartimente vorgesehen sind, in denen jeweils ein oder mehrere Konzentrate vorliegen.

8. Vorrichtung nach einem der vorhergehenden Ansprüche, **dadurch gekennzeichnet, dass** das Konzentrat in dem Behälter als Pulver, Granulat, Slurry oder in flüssiger Form vorliegt.

9. Vorrichtung nach einem der vorhergehenden Ansprüche, **dadurch gekennzeichnet, dass** in dem ersten Teil der Vorrichtung und/oder in dem zweiten Teil der Vorrichtung eine Pumpe (3; 13b) vorgesehen ist.

10. Vorrichtung nach einem der vorhergehenden Ansprüche, **dadurch gekennzeichnet, dass** in dem ersten und/oder in dem zweiten Teil der Vorrichtung ein Sensor, vorzugsweise eine Leitfähigkeitsmesszelle (5; 16) angeordnet ist.

11. Vorrichtung nach einem der vorhergehenden Ansprüche, **dadurch gekennzeichnet, dass** in den zweiten Teil der Vorrichtung eine Konzentratleitung einmündet, die mit einem weiteren Reservoir enthaltend ein Dialysatkonzentrat verbunden ist.

12. Verfahren zur Herstellung von Dialysat gemäß einer vorrichtung nach einem der Ansprüche 1 bis 11, **dadurch gekennzeichnet, dass** der Primärseite des Filters (4) Wasser zugeführt wird, dass das Permeat durch Forward-Osmose der Sekundärseite zugeführt wird und dass der Sekundärseite des Filters (4) aus dem Behälter (14) und/oder aus einer sonstigen Quelle ein Dialysatkonzentrat zugeführt wird, das mit dem Permeat gemischt wird, und dass in dem zweiten Teil der Vorrichtung mehrere Behälter (14) vorhanden sind, wobei ein Behälter (14) mit dem Dialysat oder mit dem Dialysatkonzentrat gefüllt und der andere Behälter (14) zur Verwendung in einem Dialysegerät entleert wird.

13. Verfahren nach Anspruch 12, **dadurch gekennzeichnet, dass** das Dialysat oder das Dialysatkonzentrat auf der Sekundärseite des Filters (4) im Kreislauf gefördert wird, bis die Leitfähigkeit und/oder eine Konzentration oder ein sonstiger für diese Parameter repräsentativer Parameter einem Sollwert entspricht oder in einem Sollwertbereich liegt.

14. Verfahren nach einem der Ansprüche 12 bis 13, **dadurch gekennzeichnet, dass** zur Erhöhung des osmotischen Drucks auf der Sekundärseite des Filters (4) ein physiologisch verträglicher Stoff, insbesondere Glucose, oder ein vor der Verwendung als Dialysat abzuscheidener Stoff, insbesondere magnetische Nano Eisenpartikel zugegeben werden.

## Claims

1. An apparatus for preparing dialyzate, wherein the apparatus has a first part and a second part that is configured as a circuit; wherein the first part comprises a water connection or a water container (2) as well as the primary side of a filter (4); wherein the filter (4) is configured to prepare purified water from the water through forward osmosis; and wherein the second part comprises the secondary side of the filter (4), a reservoir (14), a filtrate line that leads from the secondary side of the filter (4) to the reservoir (14), and a line leading from the reservoir (14) to the secondary side of the filter (4), with the reservoir (14) being a container having means for connecting the container to q dialysis machine, **characterized in that** a plurality of containers are provided; and **in that** a valve arrangement is present that is configured to connect the plurality of containers alternatingly in fluid communication with the second part of the apparatus.

2. An apparatus in accordance with claim 1, **characterized in that** the container is a bag that is designed with flexible walls overall or in part or is a cartridge that is designed with rigid walls overall or in part.

3. An apparatus in accordance with claim 1 or claim 2, **characterized in that** the first part of the apparatus is also designed as a circuit.

4. An apparatus in accordance with one of the preceding claims, **characterized in that** dialysis concentrate is located in the container.

5. An apparatus in accordance with one of the preceding claims, **characterized in that** the means of the container are a connector or a tube or a connector for a tube.

6. An apparatus in accordance with one of the preceding claims, **characterized in that** a bicarbonate concentrate and/or an acid concentrate is present in the container that is formed for the preparation of dialyzate.

7. An apparatus in accordance with one of the preceding claims, **characterized in that** a plurality of compartments are provided in the container in which one or more concentrates are respectively present.

8. An apparatus in accordance with one of the preceding claims, **characterized in that** the concentrate is present in the container as a powder, a granulate, a slurry, or in liquid form.

9. An apparatus in accordance with one of the preceding claims, **characterized in that** a pump (3; 13b) is provided in the first part of the apparatus and/or in the second part of the apparatus.

10. An apparatus in accordance with one of the preceding claims, **characterized in that** a sensor, preferably a conductivity measuring cell (5; 16) is arranged in the first part of the apparatus and/or in the second part of the apparatus.

11. An apparatus in accordance with one of the preceding claims, **characterized in that** a concentrate line that is centrally connected to a further reservoir containing a dialysis concentrate opens into the second part of the apparatus.

12. A method of preparing dialyzate in accordance with an apparatus in accordance with one of the claims 1 to 11, **characterized in that** water is supplied to the primary side of the filter (4); **in that** the permeate is supplied to the secondary side by forward osmosis; and **in that** a dialysis concentrate that is mixed with the permeate is supplied to the secondary side of the filter (4) from the container (14) and/or from another source; and **in that** a plurality of containers (14) are present in the second part of the apparatus, wherein one container (14) is filled with the dialyzate or with the dialysis concentrate and the other container (14) is emptied for use in a dialysis machine.

13. A method in accordance with claim 12, **characterized in that** the dialyzate or the dialysis concentrate is conveyed in the circuit on the secondary side of the filter (4) until the conductivity and/or a concentration or another parameter representative for these parameters corresponds to a desired value or is in a desired value range.

14. A method in accordance with one of the claims 12 to 13, **characterized in that** a physiologically compatible substance, in particular glucose, or a substance to be separated prior to the use as a dialyzate, in particular magnetic iron nanoparticles, are added to increase the osmotic pressure on the secondary side of the filter (4).

## Revendications

1. Dispositif de préparation de dialysat, le dispositif présentant une première partie et une seconde partie réalisée sous la forme d'un circuit, la première partie comprenant un raccordement d'eau ou un récipient d'eau (2) ainsi que le côté primaire d'un filtre (4), le filtre (4) étant conçu pour préparer, à partir de l'eau, de l'eau purifiée par osmose directe, et la seconde partie comprenant le côté secondaire du filtre (4), un réservoir (14), une conduite de filtrat qui va du côté secondaire du filtre (4) au réservoir (14) et une conduite qui va du réservoir (14) au côté secondaire du filtre (4), le réservoir (14) étant un récipient qui dispose de moyens pour relier le récipient à un appareil de dialyse, **caractérisé en ce que** plusieurs récipients sont prévus et par la présence d'un ensemble de soupapes qui est conçu pour commuter les plusieurs récipients en alternance en liaison fluidique avec la seconde partie du dispositif.

2. Dispositif selon la revendication 1, **caractérisé en ce que** le récipient est une poche qui est exécutée entièrement ou partiellement avec des parois flexibles, ou une cartouche qui est exécutée entièrement ou partiellement avec des parois rigides.

3. Dispositif selon la revendication 1 ou 2, **caractérisé en ce que** la première partie du dispositif est également exécutée sous la forme d'un circuit.

4. Dispositif selon l'une des revendications précédentes, **caractérisé en ce que** du concentré de dialysat se trouve dans le récipient.

5. Dispositif selon l'une des revendications précédentes, **caractérisé en ce que** les moyens du récipient sont un connecteur ou un tuyau ou un raccord pour un tuyau.

6. Dispositif selon l'une des revendications précédentes, **caractérisé en ce qu'**un concentré de bicarbonate et/ou un concentré d'acide, qui sont conçus pour la préparation de dialysat, se trouvent dans le récipient.

7. Dispositif selon l'une des revendications précédentes, **caractérisé en ce que** plusieurs compartiments dans lesquels se trouvent respectivement un ou plusieurs concentrés sont prévus dans le récipient.

8. Dispositif selon l'une des revendications précédentes, **caractérisé en ce que** le concentré présent dans le récipient se trouve sous la forme de poudre, de granulés, de suspension ou sous forme liquide.

9. Dispositif selon l'une des revendications précédentes, **caractérisé en ce qu'**une pompe (3 ; 13b) est prévue dans la première partie du dispositif et/ou dans la seconde partie du dispositif.

10. Dispositif selon l'une des revendications précédentes, **caractérisé en ce qu'**un capteur, de préférence une cellule de mesure de conductivité (5 ; 16), est disposé dans la première et/ou dans la seconde partie du dispositif.

11. Dispositif selon l'une des revendications précédentes, **caractérisé en ce qu'**une conduite de concentré, qui est reliée à un autre réservoir contenant un concentré de dialysat, débouche dans la seconde partie du dispositif.

12. Procédé de préparation de dialysat conformément à un dispositif selon l'une des revendications 1 à 11, **caractérisé en ce que** le côté primaire du filtre (4) est alimenté en eau, **en ce que** le côté secondaire est alimenté en perméat par osmose directe et **en ce que** le côté secondaire du filtre (4) est alimenté à partir du récipient (14) et/ou à partir d'une autre source en concentré de dialysat qui est mélangé avec le perméat, et **en ce que** la seconde partie du dispositif présente plusieurs récipients (14), un récipient (14) étant rempli avec le dialysat ou le concentré de dialysat et l'autre récipient (14) étant vidé pour l'utilisation dans un appareil de dialyse.

13. Procédé selon la revendication 12, **caractérisé en ce que** le dialysat ou le concentré de dialysat est mis en circulation du côté secondaire du filtre (4) jusqu'à ce que la conductivité et/ou une concentration ou un autre paramètre représentatif de ces paramètres correspondent à une valeur de consigne ou soient compris dans une plage de valeurs de consigne.

14. Procédé selon l'une des revendications 12 à 13, **caractérisé en ce qu'**une substance physiologiquement compatible, en particulier du glucose, ou une substance à extraire avant l'utilisation en tant que dialysat, en particulier des nanoparticules de fer magnétiques, est ajoutée pour augmenter la pression osmotique du côté secondaire du filtre (4).
